# FASCICULE DE BREVET EUROPEEN

(11) **EP 0 759 790 B1**
(45) Date de publication et mention de la délivrance du brevet: **01.07.1998**
(21) Numéro de dépôt: 95917384.0
(22) Date de dépôt: 12.04.1995
(51) Int. Cl.: A61M 15/00

(54) **INSERT DELIMITANT UNE CHAMBRE DE DOSAGE D'UN INHALATEUR**
EINSATZ ZUR BEGRENZUNG EINER DOSIERKAMMER EINES INHALATORS
INSERT DELIMITING THE DOSING CHAMBER OF AN INHALER

(30) Priorité: 20.04.1994 FR 9404711
(43) Date de publication de la demande: 05.03.1997
(73) Titulaire: VALOIS S.A., 27110 Le Neubourg (FR)
(72) Inventeur: BRUNA, Pascal, F-76000 Rouen (FR)
(74) Mandataire: Riege, Christian
(86) Numéro de dépôt international: FR9500474
(87) Numéro de publication internationale: WO9528980

(56) Documents cités:
- WO-A-90/07351
- WO-A-92/04068
- WO-A-93/18812
- DE-U- 8 703 534
- FR-A- 2 700 279

## Description

La présente invention concerne un dispositif d'inhalation de produit sous forme de poudre et plus particulièrement un insert délimitant avec précision la chambre de dosage dudit dispositif d'inhalation.

Les inhalateurs de poudre sont aujourd'hui très répandus et trouvent une de leurs applications principales dans le domaine pharmaceutique et notamment dans celui des médicaments traitant l'asthme. Un tel inhalateur est divulgué notamment dans le document EP-632 734.

Or, ces médicaments sont souvent très onéreux et doivent être dosés avec précision. De plus le dosage peut varier selon les patients mais aussi au cours d'un même traitement. Il est donc nécessaire de prévoir différents dispositifs d'inhalation selon la quantité de produit à distribuer.

Pour limiter les coûts de fabrication, et donc de vente, des dispositifs d'inhalation de poudre, il est par conséquent souhaitable de pouvoir varier très simplement, à l'assemblage, grâce à des modifications d'un minimum de pièces, la quantité de produit émis à chaque dose, c'est-à-dire le volume de la chambre de dosage.

Les documents WO-A-92/04068 et FR-2 700 279 divulguent des appareils incorporant une chambre de dosage mobile entre une position de repos, où elle est située dans le réservoir de produit, et une position d'actionnement, où elle est située dans le conduit d'expulsion. La chambre de dosage se remplit dans sa position de repos et est ensuite déplacée vers la position d'actionnement par l'utilisateur. Cette mise en oeuvre présente plusieurs inconvénients : D'une part, elle nécessite un mécanisme complexe pour déplacer la chambre de dosage, ce qui augmente le coût de fabrication de l'appareil. D'autre part, le déplacement de la chambre de dosage devant se faire relativement sans jeu latéral, des forces de frottements peuvent apparaître qui risquent d'empêcher le bon fonctionnement de l'appareil. En outre, lors du déplacement de la chambre de dosage, une partie du produit peut être perdue et la reproductibilité de la dose n'est pas garantie.

Un but de l'invention est de fournir un moyen permettant de résoudre le problème susmentionné en évitant les inconvénients précités.

La présente invention a donc pour objet un dispositif d'inhalation de produit sous forme de poudre selon la revendication 1.

Le terme "disposée fixement dans ledit conduit d'expulsion" signifie que la chambre de dosage n'est pas déplaçable hors du conduit d'expulsion, mais est au contraire disposée à tout instant dans ledit conduit d'expulsion. Bien entendu, selon sa dimension, cette chambre de dosage peut comporter des zones d'extrémité s'étendant au-delà dudit conduit d'expulsion, et elle peut également être de configuration déformable, tout en restant disposée en permanence dans le conduit d'expulsion.

De préférence, lors de l'expulsion du produit par l'air comprimé envoyé par la pompe dans le conduit d'expulsion, la chambre de dosage est obturée de manière étanche au niveau du réservoir, au moyen d'un dispositif d'étanchéité amovible situé à l'intérieur dudit réservoir.

Avantageusement, ledit insert comporte une partie inférieure indéformable fixée à une pièce support, ladite pièce support faisant partie du corps du dispositif et étant traversée par ledit conduit d'expulsion, et une partie supérieure prolongeant ladite partie inférieure et faisant saillie à l'intérieur du conduit d'expulsion de manière étanche, ladite partie supérieure comportant une paroi latérale ayant environ une forme tronconique et, dans sa partie centrale, en regard du réservoir, un évidement délimitant la chambre de dosage, la dose de produit retenue dans ladite chambre de dosage étant expulsée, lors de l'actionnement du dispositif, par l'air comprimé envoyé par la pompe à travers ledit conduit d'expulsion, en direction dudit embout de sortie.

Selon un premier mode de réalisation de l'invention, ledit insert est rigide et indéformable en totalité, sa partie supérieure saillante dans le conduit d'expulsion s'étendant sur une partie de la hauteur dudit conduit d'expulsion de sorte à préserver un passage pour l'air comprimé à l'intérieur dudit conduit d'expulsion.

Avantageusement, lequel ledit insert comporte sous ledit évidement délimitant la chambre de dosage, un plot s'étendant jusqu'à un élément fixe du dispositif et s'appuyant sur celui-ci pour renforcer la rigidité dudit insert. Cet élément fixe peut par exemple être le corps de pompe.

Un autre but de l'invention est de garantir l'étanchéité totale de la dose de produit à distribuer jusqu'au moment où elle est expulsée de l'inhalateur. En effet, un inconvénient principal de certains inhalateurs est qu'entre le moment où la chambre de dosage est chargée avec la dose de produit à émettre, et le moment où le dispositif est effectivement actionné, ladite dose de produit est située dans le conduit d'expulsion au contact de l'air. Si le dispositif n'est pas actionné immédiatement après le chargement de la chambre de dosage, le produit peut donc être contaminé, et, si le dispositif est utilisé en milieu humide, la poudre peut s'agglomérer sous l'effet de l'humidité. Le produit sera alors distribué sous forme compact et perdra ainsi une grande partie de son efficacité. La présente invention a donc également pour but de fournir un dispositif d'inhalation comportant une chambre de dosage totalement étanche jusqu'à l'actionnement du dispositif, évitant ainsi les inconvénients précités.

La présente invention a donc également pour objet, dans un second mode de réalisation, un dispositif d'inhalation comportant un insert dont la partie supérieure est déformable sous l'effet de la pression de l'air comprimé, ladite partie supérieure de l'insert s'étendant sur la totalité de la hauteur du conduit d'expulsion pour venir s'appuyer sur sa paroi supérieure et ainsi obturer celui-ci lorsque le dispositif n'est pas actionné, ladite chambre de dosage étant par conséquent isolée de manière totalement étanche, au niveau du réservoir, par un dispositif d'étanchéité amovible situé à l'intérieur du réservoir, et au niveau du conduit d'expulsion, par la partie supérieure de l'insert.

De préférence, ladite partie supérieure dudit insert comporte dans sa paroi latérale, au niveau de la jonction de la partie supérieure de l'insert avec la partie inférieure de l'insert, une zone amincie formant charnière souple au niveau de laquelle ladite partie supérieure dudit insert se déforme sous l'action de la force exercée par l'air comprimé lors de l'actionnement du dispositif, pour libérer un passage pour l'air comprimé, et reprend sa forme initiale lorsque le dispositif est au repos.

Avantageusement, ledit évidement délimitant la chambre de dosage est indéformable et se déplace en translation vers le bas, en éloignement de la paroi supérieure du conduit d'expulsion, lorsque ladite zone amincie se déforme lors de l'actionnement du dispositif, libérant ainsi ledit passage pour l'air comprimé dans ledit conduit d'expulsion, permettant l'expulsion totale du produit situé à l'intérieur de ladite chambre de dosage. Cette mise en oeuvre de l'invention permet d'assurer que l'insert reprend sa forme initiale après chaque utilisation et que la chambre de dosage conserve toujours le même volume pour distribuer toujours la même quantité de produit.

Pour éviter une déformation éventuelle dudit évidement lors de la déformation de l'insert, ladite partie supérieure de l'insert comporte sous ledit évidement, un plot s'étendant au repos jusqu'à un point situé en éloignement d'un élément fixe du dispositif, par exemple le corps de pompe, et venant buter contre celui-ci lors de l'actionnement du dispositif, limitant ainsi la déformation dudit insert et la translation dudit évidement et définissant un passage calibré pour l'air comprimé.

Avantageusement, ladite partie supérieure de l'insert comporte sur sa paroi latérale une ou plusieurs nervures ou rainures définissant avec précision l'endroit de la zone amincie destinée à se déformer lors de l'actionnement du dispositif.

Selon l'invention, ledit insert peut avoir, en coupe horizontale, une forme circulaire, oblongue ou rectangulaire.

Avantageusement, ledit insert est réalisé en une pièce et peut être fixé à ladite pièce support par emmanchement ou par encliquetage.

Pour garantir une bonne étanchéité entre l'insert et le conduit d'expulsion, il est avantageux de réaliser ledit insert en matériau suffisamment souple tel qu'un élastomère par exemple.

D'autres caractéristiques et avantages de la présente invention apparaîtront au cours de la description suivante de deux modes de réalisation de l'invention, donnés à titre d'exemples non limitatifs en regard des dessins joints.

Sur les dessins,
- la figure 1 représente une vue schématique générale du dispositif d'inhalation selon l'invention ;
- la figure 2 représente une vue agrandie de la partie du dispositif d'inhalation comprenant la chambre de dosage, selon un premier mode de réalisation de l'invention ;
- la figure 3a représente une vue semblable à celle de la figure 2 pour un second mode de réalisation de l'invention, au repos ; et
- la figure 3b représente la même vue que celle de la figure 3a, pendant le fonctionnement du dispositif.

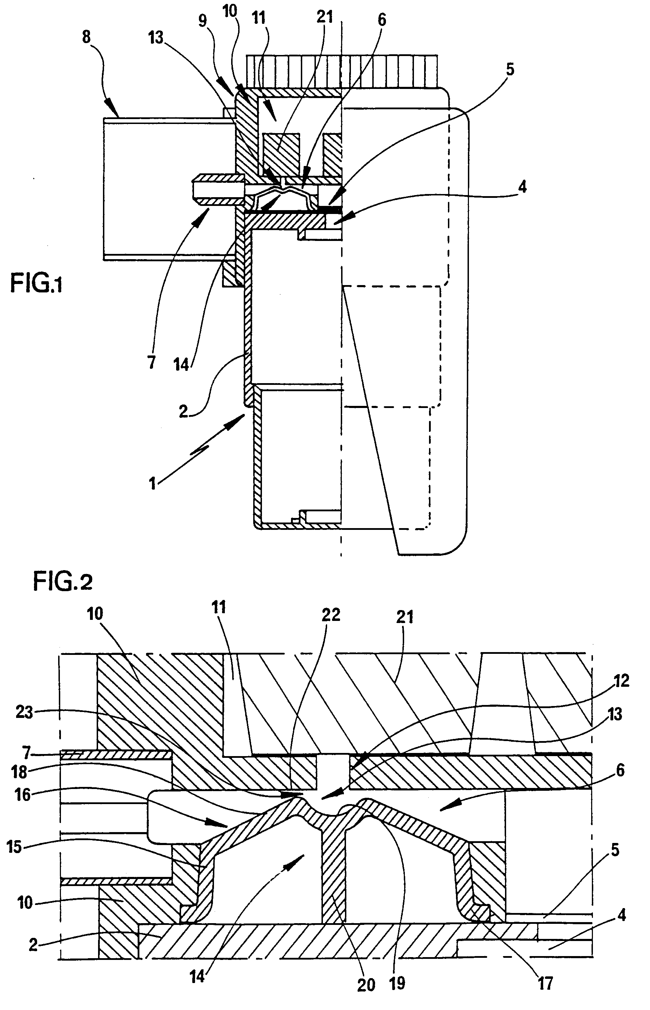

En référence à la figure 1, le dispositif d'inhalation comporte une pompe d'air comprimé 1 comprenant un corps de pompe 2 et une chambre de pompe 4, ladite pompe 1 étant destinée à expulser de l'air comprimé lorsque le dispositif est actionné. Ladite chambre de pompe 4 est reliée, via un clapet 5, à un conduit d'expulsion 6, lui-même relié à un embout de sortie 7 débouchant dans un organe d'inhalation 8 adapté à faciliter l'inhalation par le patient. Cet organe 8 peut par exemple être un embout buccal, lorsque le produit est administré par voie orale. Le dispositif comporte également un corps 9 comprenant une pièce support 10, qui supporte l'embout de sortie 7 et ledit organe d'inhalation 8, et qui est traversée par ledit conduit d'expulsion 6. Avantageusement, la pompe 1 peut être dissociée du reste du dispositif et vient s'encliqueter sur ledit corps 9 du dispositif lors de l'assemblage, au moyen dudit corps de pompe 2. Toutefois, le corps de pompe 2 et le corps 9 du dispositif peuvent également être solidaires l'un de l'autre. Le dispositif comporte de plus un réservoir de produit 11, relié audit conduit d'expulsion 6 par un petit trou 12. De préférence, le réservoir est situé au-dessus dudit conduit d'expulsion 6, comme représenté sur les figures. Ledit petit trou 12 forme une partie de la chambre de dosage 13 qui débouche dans le conduit d'expulsion 6 et qui comprend un insert 14 faisant saillie par une partie supérieure 16 à l'intérieur dudit conduit d'expulsion 6. Cet insert 14 comporte une partie inférieure 15 qui peut avantageusement être cylindrique mais qui peut également avoir en coupe horizontale une forme oblongue, ou rectangulaire. Cette partie inférieure 15 de l'insert 14 se fixe sur ladite pièce support 10 du corps 9 du dispositif, avantageusement par emmanchement ou par encliquetage au moyen de pattes 17. La partie inférieure 15 de l'insert 14 se prolonge par une partie supérieure 16 faisant saillie à l'intérieur du conduit d'expulsion 6 par une ouverture prévue à cet effet dans ledit conduit. Pour un bon fonctionnement du dispositif, l'insert 14 doit être introduit dans le conduit d'expulsion de manière étanche. Il est indispensable que la partie inférieure 15 de l'insert, située hors du conduit d'expulsion, soit indéformable et reste fixement en contact étanche avec la pièce support 10 pendant le fonctionnement du dispositif pour éviter toute fuite éventuelle. Avantageusement, l'insert 14 est réalisé en un matériau relativement souple permettant d'assurer ladite étanchéité au niveau de l'ouverture du conduit par laquelle l'insert pénètre dans ce dernier.

La chambre de dosage 13 est donc disposée à tout instant dans et juste autour du conduit d'expulsion 6 et il n'est pas nécessaire de prévoir un mécanisme complexe pour déplacer ladite chambre de dosage hors dudit conduit. On peut donc dire que la chambre de dosage 13 est disposée fixement dans ledit conduit d'expulsion 6.

D'autre part, pour assurer l'étanchéité au niveau du réservoir 11 lors du fonctionnement du dispositif, le petit trou 12 de la chambre de dosage 13 est obturé de manière étanche par un dispositif d'étanchéité amovible 21 situé à l'intérieur dudit réservoir 11. Ce dispositif d'étanchéité 21 est enlevé du trou 12 pour permettre le chargement du produit dans la chambre de dosage 13 puis remis en place en vue de l'actionnement du dispositif.

La partie supérieure 16 de l'insert 14, saillante dans le conduit d'expulsion 6, présente une forme environ tronconique et comporte une paroi latérale 18 se prolongeant à partir de ladite partie inférieure 15, ainsi que dans sa partie centrale en face du trou 12, un évidement 19 adapté à retenir une dose de produit et délimitant la chambre de dosage 13.

Un premier mode de réalisation de l'invention est représenté sur la figure 2. Dans ce mode de réalisation, l'insert 14 est rigide et indéformable dans sa totalité. La paroi latérale 18 de sa partie supérieure 16 s'étend donc uniquement sur une partie de la hauteur du conduit d'expulsion et ne vient pas s'appliquer contre la paroi supérieure 22 dudit conduit, pour préserver un passage 23 pour l'air comprimé à l'intérieur dudit conduit 6 lorsque le dispositif est actionné. Ainsi, un cycle d'expulsion d'une dose de produit se décompose dans les étapes suivantes :

Le dispositif d'étanchéité 21 situé dans le réservoir 11 est enlevé du trou 12, c'est-à-dire de la chambre de dosage 13, et cette dernière est remplie de produit. Le produit tombe donc par gravité ou avec l'aide d'un moyen extérieur, à travers le trou 12 dans l'évidement 19 de l'insert 14. Lorsque la chambre de dosage 13 (évidement 19 + trou 12) est pleine, le dispositif d'étanchéité 21 du réservoir revient boucher ledit trou 12 de manière étanche. Le dispositif est alors actionné et l'air comprimé issu de la pompe 1 passe de la chambre de pompe 4 dans le conduit d'expulsion 6 via le clapet 5 pour ensuite, par l'intermédiaire du passage 23, vider entièrement la chambre de dosage 13 du produit qu'elle contient. Ladite dose de produit est ainsi distribuée sous forme pulvérisée par l'embout de sortie 7 dans l'organe d'inhalation 8, puis au patient.

Avantageusement, la pression de l'air comprimé pouvant être élevée, la rigidité de l'insert 14 est augmentée en prévoyant un plot 20 disposé sous ledit évidement 19 de l'insert 14 et s'étendant dudit évidement 19 jusqu'a un élément fixe du dispositif, par exemple le corps de pompe 2 tel que représenté sur les figures, sur lequel il vient s'appuyer. Ce plot 20 peut être d'une pièce avec l'insert 14.

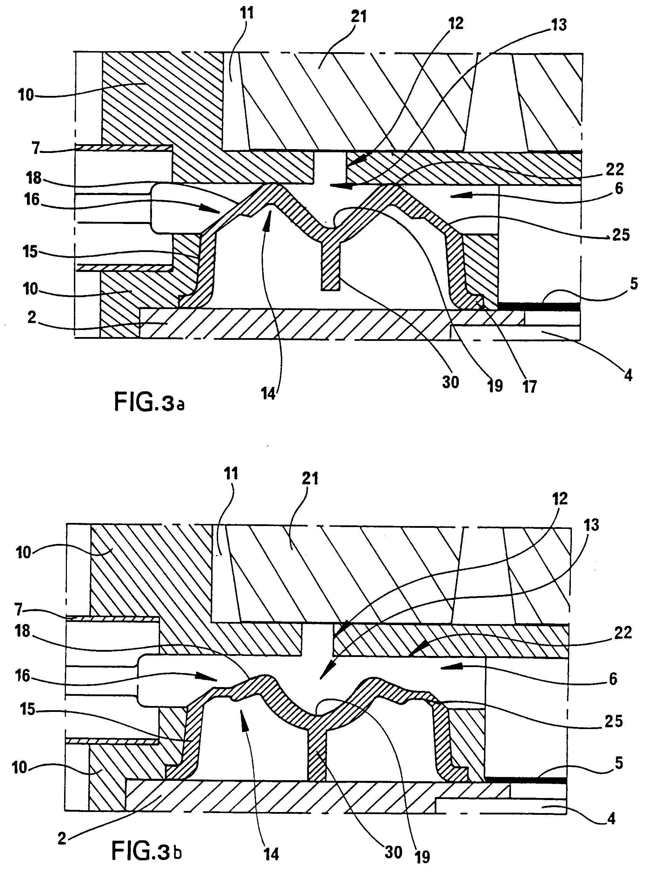

Un second mode de réalisation de l'invention est représenté sur les figures 3a et 3b. Dans ce mode de réalisation, la partie supérieure 16 de l'insert 14 est déformable sous l'effet de la pression de l'air comprimé et la paroi latérale 18 s'étend sur la totalité de la hauteur du conduit d'expulsion 6 pour venir s'appuyer sur sa paroi supérieure 22. Au repos, le conduit d'expulsion 6 est donc obturé par l'insert 14 et la chambre de dosage 13 est par conséquent totalement étanche lorsque le dispositif n'est pas actionné. En effet, au niveau du réservoir 11, l'étanchéité est assurée par le dispositif d'étanchéité amovible 21 précité disposé à l'intérieur du réservoir 11 et au niveau du conduit d'expulsion 6, l'étanchéité est donc assurée par l'insert 14. Cette étanchéité absolue empêche une contamination du produit entre le moment où la chambre de dosage 13 est chargée et le moment où elle est vidée par l'actionnement du dispositif. De même, si un certain laps de temps s'écoule entre ledit chargement et ledit vidage, le produit est préservé d'une éventuelle humidité extérieure qui pourrait restreindre l'efficacité de la dose émise en provoquant une agglomération de la poudre par exemple. Ce mode de réalisation de l'invention est par conséquent avantageux.

Comme déjà mentionné ci-dessus, la partie supérieure 16 de l'insert 14 est donc déformable sous l'effet de la pression de l'air comprimé. Ainsi, lorsque le dispositif est actionné, l'air comprimé, envoyé par la pompe 1 dans le conduit d'expulsion 6 via la chambre de pompe 4 et le clapet 5, vient buter contre la paroi latérale 18 de l'insert et occasionne la déformation de ce dernier, libérant ainsi un passage pour l'air comprimé dans le conduit d'expulsion. L'air comprimé peut donc vider entièrement la chambre de dosage 13, le produit étant transporté jusqu'à l'embout de sortie 7 puis distribué au patient par l'intermédiaire de l'organe d'inhalation 8. Il est à noter que même si une partie de la chambre de dosage 13 est déformable, cette dernière reste en permanence dans le conduit d'expulsion 6.

Avantageusement, la partie supérieure 16 de l'insert 14 comporte dans sa paroi latérale 18, au niveau de la jonction des parties supérieure 16 et inférieure 15 de l'insert 14, une zone amincie 25. Cette zone amincie 25 a pour fonction de former une sorte de charnière souple et élastique qui se plie sous l'effet de la pression de l'air comprimé pour libérer ledit passage pour l'air comprimé et qui reprend sa forme initiale dès lors qu'il n'y a plus d'air comprimé dans le conduit d'expulsion 6. De préférence, lors de la déformation de l'insert 14, l'évidement 19 délimitant la chambre de dosage 13 ne se déforme pas lui-même, et se déplace en translation en éloignement de la paroi supérieure 22 du conduit d'expulsion 6. Cette mise en oeuvre préférée de l'invention assure que le volume de produit contenu dans la chambre de dosage 13 ne varie pas, même après plusieurs actionnements du dispositif, c'est-à-dire après plusieurs déformations de l'insert 14. Pour éviter que la déformation de l'insert 14 ne soit trop importante, ce qui pourrait entraîner une déformation de l'évidement 19, on prévoit avantageusement un plot 30 sous l'évidement 19. Ce plot, généralement d'une pièce avec l'insert 14, s'étend au repos jusqu'à un point situé en éloignement d'un élément fixe du dispositif, par exemple le corps de pompe 2 tel que représenté sur les figures, et vient buter contre celui-ci lors de l'actionnement du dispositif, limitant ainsi la déformation de l'insert 14 et la translation de l'évidement 19, ledit plot 30 définit en outre un passage calibré pour l'air comprimé, assurant ainsi une distribution identique de chaque dose de produit.

Pour déterminer de manière précise l'endroit où la zone amincie 25 de l'insert 14 doit se déformer lors de l'actionnement du dispositif, on peut prévoir sur la paroi latérale 18 de l'insert une ou plusieurs rainures ou nervures (non représentées).

Avantageusement, l'insert 14 selon l'invention est réalisé en une pièce et peut être en élastomère ou tout autre matériau suffisamment souple, la déformation dudit insert pouvant être due soit au matériau constituant l'insert, soit à la forme dudit insert, tel que décrit précédemment.

## Revendications

1. Dispositif d'inhalation de produit sous forme de poudre comprenant un corps (9), une pompe d'air comprimé (1) comportant un corps de pompe (2) et une chambre de pompe (4), un conduit d'expulsion (6) relié à la chambre de pompe (4) et débouchant dans un embout de sortie (7) par lequel le produit est distribué, un réservoir de produit (11) relié audit conduit d'expulsion (6), et une chambre de dosage de produit (13), caractérisé en ce que ladite chambre de dosage (13) comporte un insert (14) disposé fixement par rapport au conduit d'expulsion (6) et faisant saillie, par une partie supérieure (16), à l'intérieur dudit conduit d'expulsion (6), de manière étanche à travers une ouverture prévue à cet effet dans ledit conduit (6), ladite partie supérieure (16) dudit insert (14) comportant un profil (19) adapté à retenir une dose prédéterminée de produit, ladite chambre de dosage étant à tout instant disposée dans ledit conduit d'expulsion et n'est pas déplaçable hors du conduit d'expulsion et en ce que ledit conduit d'expulsion (6) est relié à la chambre de pompe (4) via un clapet (5).

2. Dispositif d'inhalation selon la revendication 1, dans lequel, lors de l'expulsion du produit par l'air comprimé envoyé par la pompe (1) dans le conduit d'expulsion (6), la chambre de dosage (13) est obturée de manière étanche au niveau du réservoir (11), au moyen d'un dispositif d'étanchéité amovible (21) situé à l'intérieur dudit réservoir (11).

3. Dispositif d'inhalation selon la revendication 1 ou 2, dans lequel ledit insert (14) comporte une partie inférieure (15) indéformable fixée à une pièce support (10), ladite pièce support faisant partie du corps (9) du dispositif et étant traversée par ledit conduit d'expulsion (6), et une partie supérieure (16) prolongeant ladite partie inférieure (15) et faisant saillie à l'intérieur du conduit d'expulsion (6) de manière étanche, ladite partie supérieure (16) comportant une paroi latérale (18) ayant environ une forme tronconique et, dans sa partie centrale, en regard du réservoir (11), un évidement (19) délimitant la chambre de dosage (13), la dose de produit retenue dans ladite chambre de dosage (13) étant expulsée, lors de l'actionnement du dispositif, par l'air comprimé envoyé par la pompe (1) à travers ledit conduit d'expulsion (6), en direction dudit embout de sortie (7).

4. Dispositif d'inhalation selon l'une quelconque des revendications précédentes, dans lequel ledit insert (14) est rigide et indéformable en totalité, sa partie supérieure (16) saillante dans le conduit d'expulsion (6) s'étendant sur une partie de la hauteur dudit conduit d'expulsion (6) de sorte à préserver un passage (23) pour l'air comprimé à l'intérieur dudit conduit d'expulsion (6).

5. Dispositif d'inhalation selon les revendications 3 et 4, dans lequel ledit insert (14) comporte sous ledit évidement (19) délimitant la chambre de dosage (13), un plot (20) s'étendant jusqu'à un élément fixe du dispositif et s'appuyant sur celui-ci pour renforcer la rigidité dudit insert (14).

6. Dispositif d'inhalation selon la revendication 5, dans lequel ledit élément fixe du dispositif est le corps de pompe (2).

7. Dispositif d'inhalation selon l'une quelconque des revendications 1 à 3, dans lequel ladite partie supérieure (16) dudit insert (14) est déformable sous l'effet de la pression de l'air comprimé, ladite partie supérieure (16) de l'insert (14) s'étendant sur la totalité de la hauteur du conduit d'expulsion (6) pour venir s'appuyer sur sa paroi supérieure (22) et ainsi obturer celui-ci lorsque le dispositif n'est pas actionné, ladite chambre de dosage (13) étant par conséquent isolée de manière totalement étanche, au niveau du réservoir (11), par un dispositif d'étanchéité amovible (21) situé à l'intérieur du réservoir (11), et au niveau du conduit d'expulsion (6), par la partie supérieure (16) de l'insert (14).

8. Dispositif d'inhalation selon la revendication 7, dans lequel ladite partie supérieure (16) dudit insert (14) comporte dans sa paroi latérale (18), au niveau de la jonction de la partie supérieure (16) de l'insert (14) avec la partie inférieure (15) de l'insert (14), une zone amincie (25) formant charnière souple, au niveau de laquelle ladite partie supérieure (16) dudit insert (14) se déforme sous l'action de la force exercée par l'air comprimé lors de l'actionnement du dispositif, pour libérer un passage pour l'air comprimé, et reprend sa forme initiale lorsque le dispositif est au repos.

9. Dispositif d'inhalation selon la revendication 8, dans lequel ledit évidement (19) délimitant la chambre de dosage (13) est indéformable et se déplace en translation vers le bas, en éloignement de la paroi supérieure (22) du conduit d'expulsion (6), lorsque ladite zone amincie (25) se déforme lors de l'actionnement du dispositif, libérant ainsi ledit passage pour l'air comprimé dans ledit conduit d'expulsion (6), permettant l'expulsion totale du produit situé à l'intérieur de ladite chambre de dosage (13).

10. Dispositif d'inhalation selon la revendication 9, dans lequel ladite partie supérieure (16) de l'insert (14) comporte sous ledit évidement (19), un plot (30) s'étendant au repos jusqu'à un point situé en éloignement d'un élément fixe du dispositif, et venant buter contre celui-ci lors de l'actionnement du dispositif, limitant ainsi la déformation dudit insert (14) et la translation dudit évidement (19) et définissant un passage calibré pour l'air comprimé.

11. Dispositif d'inhalation selon la revendication 10, dans lequel ledit élément fixe du dispositif est le corps de pompe (2).

12. Dispositif d'inhalation selon l'une quelconque des revendications 8 à 11, dans lequel ladite partie supérieure (16) de l'insert (14) comporte sur sa paroi latérale (18) une ou plusieurs nervures ou rainures définissant avec précision l'endroit de la zone amincie destinée à se déformer lors de l'actionnement du dispositif.

13. Dispositif d'inhalation selon l'une quelconque des revendications précédentes, dans lequel ledit insert (14) est réalisé en une pièce.

14. Dispositif d'inhalation selon l'une quelconque des revendications précédentes, dans lequel ledit insert (14) est fixé à ladite pièce support (10) par emmanchement ou par encliquetage.

15. Dispositif d'inhalation selon l'une quelconque des revendications précédentes, dans lequel ledit insert (14) est réalisé en un matériau souple.

16. Dispositif d'inhalation selon l'une quelconque des revendications précédentes, dans lequel ledit insert (14) a, en coupe horizontale, une forme oblongue.

## Claims

1. An inhaler device for inhaling substance in powder form, the device comprising a body (9), a compressed air pump (1) including a pump body (2) and a pump chamber (4), an expulsion duct (6) connected to the pump chamber (4) and opening out into an outlet endpiece (7) through which the substance is dispensed, a substance reservoir (11) connected to said expulsion duct (6), and a substance measuring chamber (13), the device being characterized in that said measuring chamber (13) comprises an insert (14) disposed fixedly relative to the expulsion duct (6) and having a top portion (16) projecting into said expulsion duct (6) in sealed manner through an opening provided for that purpose in said duct (6), said top portion (16) of said insert (14) being of a shape (19) that is suitable for retaining a predetermined quantity of substance, said measuring chamber being located at all times in said expulsion duct and being incapable of being displaced out from the expulsion duct, and in that said expulsion duct (6) is connected to the pump chamber (4) via a valve (5).

2. An inhaler device according to claim 1, in which, while the substance is being expelled by the compressed air delivered by the pump (1) to said expulsion duct (6), the measuring chamber (13) is closed in sealed manner from the reservoir (11) by means of a movable sealing device (21) situated inside said reservoir (11).

3. An inhaler device according to claim 1 or 2, in which said insert (14) includes an undeformable bottom portion (15) fixed to a support part (10), said support part being integral with the body (9) of the device and having said expulsion duct (6) passing therethrough, and a top portion (16) extending said bottom portion (15) and projecting into the expulsion duct (6) in sealed manner, said top portion (16) including a side wall (18) that is approximately frustoconical in shape and that includes a recess (19) in its central portion facing the reservoir (11) to define the measuring chamber (13), the quantity of substance retained in said measuring chamber (13) being expelled, on actuation of the device, along said expulsion duct (6) towards said outlet endpiece (7) by the compressed air delivered by the pump (1).

4. An inhaler device according to any preceding claim, in which the entire insert (14) is rigid and undeformable, its top portion (16) projecting into the expulsion duct (6) extending over a fraction of the height of said expulsion duct (6) so as to keep a passage (23) for compressed air along said expulsion duct (6).

5. An inhaler device according to claims 3 and 4, in which said insert (14) includes a stud (20) beneath said recess (19) defining the measuring chamber (13), the stud (20) extending to a fixed element of the device and bearing thereagainst to reinforce the rigidity of said insert (14).

6. An inhaler device according to claim 5, in which said fixed element of the device is the pump body (2).

7. An inhaler device according to any one of claims 1 to 3, in which said top portion (16) of said insert (14) is deformable under the effect of the pressure of the compressed air, said top portion (16) of the insert (14) extending over the entire height of the expulsion duct (6) to press against the top wall (22) thereof and thus close the duct when the device is not actuated, said measuring chamber (13) consequently being isolated in completely sealed manner from the reservoir (11) by a movable sealing device (21) situated inside the reservoir (10), and from the expulsion duct (6) by the top portion (16) of the insert (14).

8. An inhaler device according to claim 7, in which said top proton (16) of said insert (14) includes a thin zone (25) in its side wall (18) where the top portion (16) of the insert (14) joins the bottom portion (15) of the insert (14), the thin zone (25) forming a flexible hinge where said top portion (16) of said insert (14) deforms under the action of the force exerted by the compressed air during actuation of the device to release a passage for the compressed air, and returns to its initial shape when the device is at rest.

9. An inhaler device according to claim 8, in which said recess (19) defining the measuring chamber (13) is undeformable and moves downwards in translation away from the top wall (22) of the expulsion duct (6) when said thin zone (25) deforms during actuation of the device, thereby releasing said passage for the compressed air along said expulsion duct (6), enabling all of the substance situated inside said measuring chamber (13) to be expelled.

10. An inhaler device according to claim 9, in which said top portion (16) of the insert (14) includes a stud (30) beneath said recess (19) and extending at rest to a point that is situated at a distance from a fixed element of the device, and coming into abutment thereagainst during actuation of the device, thereby limiting deformation of said insert (14) and limiting translation of said recess (19), thus defining a calibrated passage for the compressed air.

11. An inhaler device according to claim 10, in which said fixed element of the device is the pump body (2).

12. An inhaler device according to any one of claims 8 to 11, in which said top portion (16) of the insert (14) includes one or more ribs or grooves in its side wall (18), thereby accurately defining the location where the thin zone is to deform during actuation of the device.

13. An inhaler device according to any preceding claim, in which said insert (14) is made as a single piece.

14. An inhaler device according to any preceding claim, in which said insert (14) is fixed to said support part (10) by mutual engagement or by snap-fastening.

15. An inhaler device according to any preceding claim, in which said insert (14) is made of flexible material.

16. An inhaler device according to any preceding claim, in which said insert (14) is oblong in shape in horizontal section.

## Patentansprüche

1. Inhalationsvorrichtung für ein pulverförmiges Produkt, die einen Körper (9), eine Druckluftpumpe (1) mit einem Pumpenkörper (2) und einer Pumpenkammer (4), eine Ausstoßleitung (6), die mit der Pumpenkammer (4) verbunden ist und in einer Ausgangsöffnung (7) mündet, durch die das Produkt abgegeben wird, einen mit der Ausstoßleitung (6) verbundenen Vorratsbehälter (11) für das Produkt und eine Dosierkammer (13) für das Produkt umfaßt, dadurch gekennzeichnet, daß die Dosierkammer (13) einen Einsatz (14) umfaßt, der fest bezüglich der Ausstoßleitung (6) angeordnet ist und mit einem oberen Teil (16) in das Innere der Ausstoßleitung (6) in dichter Weise durch eine zu diesem Zweck in der Leitung (6) vorgesehene Öffnung hindurch vorsteht, wobei der obere Teil (16) des Einsatzes (14) ein Profil (19) aufweist, das geeignet ist, eine vorbestimmte Dosis des Produktes zurückzuhalten, wobei die Dosierkammer zu jedem Zeitpunkt innerhalb der Ausstoßleitung angeordnet und nicht über die Ausstoßleitung hinaus verschiebbar ist, und daß die Ausstoßleitung (6) mit der Pumpenkammer (4) über ein Klappenventil (5) verbunden ist.

2. Inhalationsvorrichtung nach Anspruch 1, bei der während des Ausstossens des Produktes durch von der Pumpe (1) in der Ausstoßleitung (6) abgegebene Druckluft die Dosierkammer (13) in dichter Weise im Bereich des Behälters (11) vermittels einer bewegbaren Dichtvorrichtung (21) verschlossen ist, die im Inneren des Behälters (11) angeordnet ist.

3. Inhalationsvorrichtung nach Anspruch 1 oder 2, bei der der Einsatz (14) einen unteren, nicht verformbaren Teil (15) aufweist, der an einem Tragelement (10) befestigt ist, wobei das Tragelement einen Teil des Körpers (9) der Vorrichtung bildet und von der Ausstoßleitung (6) durchzogen wird, sowie einen oberen Teil (16), der den unteren Teil (15) verlängert und in das Innere der Ausstoßleitung (6) in dichter Weise vorspringt, wobei der obere Teil (16) eine Seitenwand (18) umfaßt, die in etwa eine stumpfkegelige Form besitzt und in ihrem mittleren Teil gegenüber dem Behälter (11) eine Vertiefung (19) umfaßt, die die Dosierkammer (13) begrenzt, wobei die Dosis des in der Dosierkammer (13) zurückgehaltenen Produktes bei der Betätigung der Vorrichtung durch komprimierte Luft, die von der Pumpe (1) auf den Weg geschickt wird, durch die Ausstoßleitung (6) in Richtung der Ausgangsöffnung (7) ausgestoßen wird.

4. Inhalationsvorrichtung nach einem der vorausgehenden Ansprüche, bei der der Einsatz (14) in seiner Gesamtheit starr und nicht verformbar ist, wobei sich sein oberer Teil (16), der in die Ausstoßleitung (6) vorspringt, über einen Teil der Höhe der Ausstoßleitung (6) in der Weise erstreckt, daß ein Durchgang (23) für komprimierte Luft im Inneren der Ausstoßleitung (6) freigelassen wird.

5. Inhalationsvorrichtung nach den Ansprüchen 3 und 4, bei der der Einsatz (14) unter der Vertiefung (19), die die Dosierkammer (13) begrenzt, einen Ansatz (20) umfaßt, der sich bis zu einem festen Element der Vorrichtung erstreckt und sich an diesem abstützt, um die Steifheit des Einsatzes (14) zu verstärken.

6. Inhalationsvorrichtung nach Anspruch 5, bei der das feste Element der Vorrichtung der Pumpenkörper (2) ist.

7. Inhalationsvorrichtung nach einem der Ansprüche 1 bis 3, bei der der obere Teil (16) des Einsatzes (14) unter der Wirkung des Drucks der Druckluft verformbar ist, wobei sich der obere Teil (16) des Einsatzes (14) über die gesamte Höhe der Ausstoßleitung (6) so erstreckt, daß er an deren oberen Wand (22) zur Anlage kommt und auf diese Weise die Leitung verschließt, wenn die Vorrichtung nicht betätigt wird, so daß folglich die Dosierkammer (13) in völlig dichter Weise im Bereich des Behälters (11) durch die bewegliche Dichtvorrichtung (21) isoliert ist, die sich im Inneren des Behälters (11) befindet, und im Bereich der Ausstoßleitung (6) durch den oberen Teil (16) des Einsatzes (14).

8. Inhalationsvorrichtung nach Anspruch 7, bei der der obere Teil (16) des Einsatzes (14) in seiner Seitenwand (18) im Bereich der Verbindung des oberen Teils (16) des Einsatzes (14) mit dem unteren Teil (15) des Einsatzes (14) eine verjüngte Zone (25) umfaßt, die ein elastisches Gelenk bildet, in dessen Bereich sich der obere Teil (16) des Einsatzes (14) unter der Wirkung der Kraft verformt, die von der Druckluft bei Betätigung der Vorrichtung ausgeübt wird, um einen Durchgang für die komprimierte Luft freizugeben, und wieder seine Ausgangsform annimmt, wenn sich die Vorrichtung in Ruhe befindet.

9. Inhalationsvorrichtung nach Anspruch 8, bei der die Vertiefung (19), welche die Dosierkammer (13) begrenzt, nicht verformbar ist und sich translatorisch nach unten verschiebt, wobei sie sich von der oberen Wand (22) der Ausstoßleitung (6) wegbewegt, wenn sich die verjüngte Zone (25) bei der Betätigung der Vorrichtung verformt und auf diese Weise einen Durchgang für komprimierte Luft in der Ausstoßleitung (6) freigibt, wodurch der vollständige Ausstoß des Produktes ermöglicht wird, das sich im Inneren der Dosierkammer (13) befindet.

10. Inhalationsvorrichtung nach Anspruch 9, bei der der obere Teil (16) des Einsatzes (14) unterhalb der Vertiefung (19) einen Ansatz (30) umfaßt, der sich in Ruhelage bis zu einem Punkt erstreckt, der von einem festen Element der Vorrichtung einen Abstand besitzt, und an diesem bei Betätigung der Vorrichtung zum Anschlag kommt, und auf diese Weise die Verformung des Einsatzes (14) und die Translationsbewegung der Vertiefung (19) begrenzt, und auf diese Weise einen genau bestimmten Durchgang für die Druckluft definiert.

11. Inhalationsvorrichtung nach Anspruch 10, bei der das feste Element der Vorrichtung der Pumpenkörper (2) ist.

12. Inhalationsvorrichtung nach einem der Ansprüche 8 bis 11, bei der der obere Teil (16) des Einsatzes (14) an seiner Seitenwand (18) eine oder mehrere Rippen oder Rillen aufweist, die sehr genau die Stelle der verjüngten Zone definieren, die dazu bestimmt ist, sich bei der Betätigung der Vorrichtung zu verformen.

13. Inhalationsvorrichtung nach einem der vorhergehenden Ansprüche, bei der der Einsatz (14) einstückig ausgebildet ist.

14. Inhalationsvorrichtung nach einem der vorhergehenden Ansprüche, bei der der Einsatz (14) an dem Tragelement (10) durch Einpressen oder Einrasten befestigt ist.

15. Inhalationsvorrichtung nach einem der vorhergehenden Ansprüche, bei der der Einsatz (14) aus einem nachgiebigen Material hergestellt ist.

16. Inhalationsvorrichtung nach einem der vorhergehenden Ansprüche, bei der der Einsatz (14) im Horizontalschnitt eine längliche Form besitzt.
